(19) Europäisches Patentamt
European Patent Office
Office européen des brevets

(11) Veröffentlichungsnummer: **0 025 010**
**B1**

(12) **EUROPÄISCHE PATENTSCHRIFT**

(45) Veröffentlichungstag der Patentschrift: **20.07.83**

(21) Anmeldenummer: **80810213.1**

(22) Anmeldetag: **27.06.80**

(51) Int. Cl.³: **C 07 C 157/00**, C 07 C 157/09, C 07 C 157/14, A 01 N 47/28, A 01 N 47/42

(54) **Thioharnstoffderivate und Isothioharnstoffderivate, Verfahren zu ihrer Herstellung, diese Verbindungen enthaltende Mittel und ihre Verwendung zur Bekämpfung von Schädlingen.**

(30) Priorität: **03.07.79 CH 6197/79**
**11.06.80 CH 4490/80**

(43) Veröffentlichungstag der Anmeldung: **11.03.81 Patentblatt 81/10**

(45) Bekanntmachung des Hinweises auf die Patenterteilung: **20.07.83 Patentblatt 83/29**

(84) Benannte Vertragsstaaten: **BE CH DE FR GB IT LI NL**

(56) Entgegenhaltungen:
**DE-A-2 143 838**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Patentabteilung Postfach, CH-4002 Basel (CH)**

(72) Erfinder: **Böger, Manfred, Lindenstrasse 33, D-7858 Weil am Rhein 5 (DE)**
Erfinder: **Drabek, Jozef, Dr., Benkenstrasse 12, CH-4104 Oberwil (CH)**

**EP 0 025 010 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Thioharnstoffderivate und Isothioharnstoffderivate, Verfahren zu ihrer Herstellung, diese Verbindungen enthaltende Mittel und ihre Verwendung zur Bekämpfung von Schädlingen

Die vorliegende Erfindung betrifft neue Thioharnstoffderivate mit pestizider Wirkung, Verfahren zu ihrer Herstellung, Schädlingsbekämpfungsmittel, welche diese Verbindungen als aktive Komponente enthalten, sowie Verfahren zur Bekämpfung von Schädlingen unter Verwendung der neuen Verbindungen.

N-Phenyl-N'-alkyl-S-alkyl-isothioharnstoffe mit pestiziden Eigenschaften sind bekannt (vgl. deutsche Offenlegungsschrift Nr. 2 730 620). Nach vorliegender Erfindung werden neuartige Thioharnstoffderivate vorgeschlagen, welche eine hohe Wirksamkeit gegen Schädlinge, vor allem gegen Vertreter der Ordnung Akarina und gegen Insekten, aufweisen, und welche aufgrund ihrer vorteilhaften biologischen Eigenschaften für die praktische Anwendung besonders geeignet sind.

Die erfindungsgemäss vorgeschlagen neuen substituierten N-Phenoxyphenyl-isothioharnstoffe entsprechen der Formel I

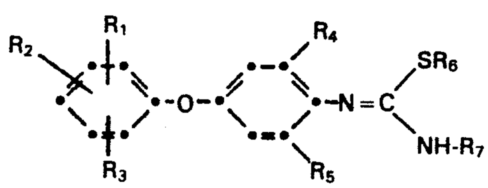

(I)

worin $R_1$, $R_2$ und $R_3$ unabhängig voneinander Wasserstoff, $C_1$-$C_4$-Alkyl, Methoxy, Halogen. Trifluormethyl oder Nitro, $R_4$ Wasserstoff oder Methyl, $R_5$ $C_1$-$C_3$-Alkyl, $R_6$ $C_1$-$C_6$-Alkyl, $C_2$-$C_6$-Alkenyl, $C_3$-$C_5$-Alkinyl, $C_3$-$C_6$-Cycloalkyl, $C_2$-$C_4$-(Alkoxyalkyl) oder $C_2$-$C_4$-(Alkylthioalkyl) und $R_7$ $C_1$-$C_{12}$-Alkyl, $C_3$-$C_5$-Alkenyl oder $C_3$-$C_8$-Cycloalkyl bedeuten.

Alkyl- bzw. Alkenyl- und Alkinylgruppen in Formel I können verzweigt oder geradkettig sein. Unter «Halogen» sind Chlor, Fluor, Brom und Jod, vorzugsweise Chlor, zu verstehen.

Die Verbindungen der Formel I können in Form von Additionssalzen anorganischer oder organischer Säuren vorliegen und erfindungsgemäss auch in Form ihrer Salze verwendet werden. Unter den Verbindungen der Formel I sind im Sinne der vorliegenden Erfindung demzufolge sowohl die freien Verbindungen der Formel I als auch deren Säureadditionssalze zu verstehen.

Die Verbindungen der Formel I können nach an sich bekannten Arbeitsweisen in ihre Säuresalze überführt werden. Zur Bildung von Additionssalzen sind beispielsweise Chlorwasserstoffsäure, Bromwasserstoffsäure, Jodwasserstoffsäure, Salpetersäure, Phosphorsäure, Schwefelsäure, Essigsäure, Propionsäure, Buttersäure, Valeriansäure, Oxalsäure, Malonsäure, Bernsteinsäure, Apfelsäure, Maleinsäure, Fumarsäure, Milchsäure, Weinsäure, Zitronensäure, Benzoesäure, Phthalsäure, Zimtsäure und Salizylsäure geeignet.

Die Verbindungen der Formel I werden analog bekannten Verfahren hergestellt, indem man z.B. einen Thioharnstoff der Formel II

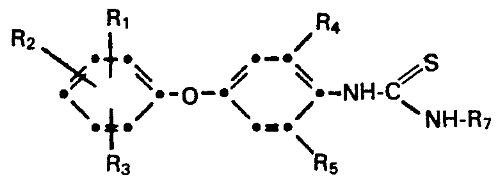

(II)

mit einem Halogenid der Formel III

$$R_6 - Hal \qquad (III)$$

umsetzt, wobei in den Formeln II und III die Reste $R_1$ bis $R_7$ die für Formel I bereits angegebenen Bedeutungen haben und «Hal» für ein Halogenatom, insbesondere ein Chlor- oder Bromatom, steht.

Das obige Verfahren wird zweckmässig bei einer Temperatur zwischen 0° und 100°C, bei normalem oder leicht erhöhtem Druck und vorzugsweise in Gegenwart eines gegenüber den Reaktionsteilnehmern inerten Lösungs- oder Verdünnungsmittels durchgeführt. Als Lösungs- oder Verdünnungsmittel eignen sich z.B. Äther und ätherartige Verbindungen, wie Di-äthyläther, Di-isopropyläther, Dioxan und Tetrahydrofuran; aromatische Kohlenwasserstoffe, wie Benzol, Toluol und Xylole; Ketone, wie Aceton, Methyläthylketon und Cyclohexanon; Alkohole und Dimethylformamid.

Die für das vorstehend beschrieben Verfahren als Ausgangsstoffe einzusetzenden Verbindungen der Formel II sind neu und bilden ebenfalls einen Gegenstand der Erfindung. Sie können aus bekannten Vorläufern leicht erhalten werden, indem man z.B. ein Isothiocyanat der Formel IV

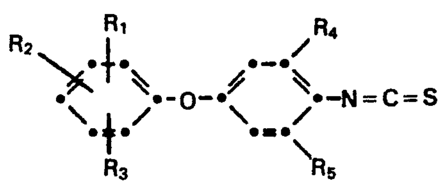

(IV)

mit einem Amin der Formel V

$$R_7 - NH_2 \qquad (V)$$

umsetzt, wobei in den Formeln IV und V $R_1$ bis $R_5$ und $R_7$ die bereits erwähnten Bedeutungen haben.

Das Verfahren zur Herstellung der Verbindungen der Formel II wird vorzugsweise in Gegenwart eines gegenüber den Reaktionsteilnehmern inerten Lösungs- oder Verdünnungsmittels, bei einer Reaktionstemperatur von 0° bis 100° unter normalem Druck durchgeführt. Als für dieses Verfahren geeignete Lösungs- und Verdünnungsmittel kommen die für das Verfahren zur Herstellung der Verbindungen der Formel I bereits erwähnten Substanzen in Betracht.

Es wurde nun überraschenderweise gefunden, dass die erfindungsgemässen Verbindungen der Formeln I und II eine besonders starke Wirkung gegen pflanzenschädigende Akariden (Milben: z.B. der Familien Tetranychidae, Tarsonemidae. Eriophydae,

Tyroglyphidae und Glycyphagidae) und auch gegen ektoparasitäre Akariden (Milben und Zecken: z.B. der Familien Ixodidae, Argasidae, Sarcoptidae und Dermanyssidae), welche Nutztiere befallen, aufweisen. Einige der erfindungsgemässen Verbindungen zeichen sich durch gute akarizidovizide Aktivität und Blattpenetrationswirkung aus. Die erfindungsgemässen Verbindungen eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsomemus pallidus, Phyllocoptes vitis und Phyllocoptura oleivora.

Darüberhinaus wurde festgestellt, dass die Verbindungen der Formeln I und II auch ausgeprägte insektizide Eigenschaften besitzen und demzufolge zur Bekämpfung von pflanzenschädigungen bzw. ektoparasitären Insekten, z.B. der Ordnungen Lepidoptera, Coleoptera, Heteroptera, Diptera, Orthoptera und Homoptera, besonders geeignet sind. Die erfindungsgemässen Verbindungen eignen sich speziell zur Bekämpfung von Blattläusen und pflanzenschädigenden Frassinsekten in Zier- und Nutzpflanzungen, wie z.B. Spodoptera littoralis und Heliothis virescens.

Wegen ihrer Wirksamkeit werden erfindungsgemäss Verbindungen der Formeln I und II bevorzugt, worin $R_1$ und $R_2$ unabhängig voneinander Wasserstoff, Methyl, Chlor, Trifluormethyl, Methoxy oder Nitro, $R_3$ Wasserstoff, $R_4$ Wasserstoff oder Methyl und $R_5$ Methyl bedeuten. Von Bedeutung sind ebenfalls diejenigen Verbindungen der Formeln I und II, worin $R_2$ Wasserstoff oder Chlor bedeutet, sowie solche, bei denen der Rest $R_1$ in der 4-Stellung steht. Hervorzuheben sind aufgrund ihrer guten pestiziden Wirkung ferner die Verbindungen der Formeln I und II, die dadurch gekennzeichnet sind, dass $R_7$ $C_1$-$C_4$-Alkyl, vorzugsweise tert.-Butyl bedeutet. Weiterhin bevorzugt sind Verbindungen der Formel I, worin $R_6$ $C_1$-$C_4$-Alkyl, vorzugsweise Methyl oder Äthyl, bedeuten.

Die Verbindungen der Formeln I und II werden erfindungsgemäss als solche verwendet oder bilden eine aktive Komponente in Mitteln, welche noch übliche geeignete Trägerstoffe oder Zuschlagstoffe oder Gemische solcher Stoffe enthalten.

Geeignete Träger- und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln.

Die insektizide bzw. akarizide Wirkung der erfindungsgemässen Mittel lässt sich durch Zusatz anderer Akarizide und/oder Insektizide wesentlich verbreitern.

Als Zusätze eigen sich z.B.: org. Phosphorverbindungen; Nitrophenole und deren Derivate; Formamidine; Harnstoffe; pyrethrinartige Verbindungen; Carbamate und chlorierte Kohlenwasserstoffe.

Die erfindungsgemässen Mittel können z.B. als Stäubemittel, Granulate, Dispersionen, Lösungen und Aufschlämmungen sowie als in Wasser dispergierbare Spritzpulver, Pasten, Emulsionen und Emulsionskonzentrate vorliegen und angewendet werden.

Der Gehalt an Wirkstoff (Verbindungen der Formeln I oder II) in den oben beschriebenen Mitteln liegt zwischen 0,1 bis 95%; dabei ist es zu erwähnen, dass bei der Applikation aus dem Flugzeug oder mittels anderer geeigneter Applikationsgeräte auch höhere Konzentrationen eingesetzt werden können.

Die Verbindungen der Formel I können beispielsweise wie folgt formuliert werden:

*Emulsionskonzentrat I*

20 Gew.-Teile des Wirkstoffes werden in
70 Gew.-Teilen Xylol gelöst und mit
10 Gew.-Teilen eines Emulgiermittels, bestehend aus einem Gemisch eines Arylphenylpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion.

*Emulsionskonzentrat II*

5 bis max. 30 Gew.-Teile Wirkstoff werden unter Rühren bei Zimmertemperatur in
30 Gew.-Teilen Dibutylphthalat
10 Gew.-Teilen Solvent 200 (niederviskoses hocharomat. Erdöldestillat)
15 bis 35 Gew.-Teilen Dutrex 238 FC (viskoses hocharomat. Erdöldestillat) gelöst und mit
10 Gew.-Teilen eines Emulgatorgemisches, bestehend aus Rizinusölpolyglykoläther und dem Calciumsalz der Dodecylbenzolsulfonsäure, versetzt. Das so erhaltene Emulsionskonzentrat gibt in Wasser milchige Emulsionen.

*Spritzpulver*

5 bis 30 Gew.-Teile des Wirkstoffes werden in einer Mischapparatur mit
5 Gew.-Teilen eines aufsaugenden Trägermaterials (Kieselsäure K 320 oder Wessalon S) und
55 bis 80 Gew.-Teilen eines Trägermaterials (Bolus alba oder Kaolin B 24) und einem Dispergiermittelgemisch, bestehend aus
5 Gew.-Teilen eines Na-lauryl-sulfonates und
5 Gew.-Teilen eines Alkyl-aryl-polyglykoläthers, intensiv vermischt. Diese Mischung wird auf einer Stift- oder Luftstrahlmühle bis auf 5-15 µm gemahlen. Das so erhaltene Spritzpulver gibt in Wasser eine gute Suspension.

*Stäubemittel:*

5 Gew.Teile feingemahlener Wirkstoff werden mit
2 Gew.-Teilen einer gefällten Kieselsäure und
93 Gew.-Teilen Talk intensiv gemischt.

*Four-on-Lösung*

| | |
|---|---|
| Wirksubstanz | 30,0 g |
| Natrium-dioctylsulfo-succinat | 3,0 g |
| Benzylalkohol | 48,0 g |
| Erdnussöl | 19,8 g |
| | 100,8 g = 100 ml |

Die Wirksubstanz wird in dem Benzylalkohol unter Rühren, eventuell auch unter leichtem Erwärmen, gelöst. Zu der Lösung wird das Natrium-dioctylsulfosuccinat und das Erdnussöl gegeben und unter Erwärmen und gründlichem Durchmischen gelöst.

*Beispiel 1*

*Herstellung von N-2,6-Dimethyl-4-(4'-trifluor methylphenoxy)-phenyl-N'-t.-butyl-S-methyl-isothioharnstoff*

Zu einer Lösung von 7,3 g N-2,6-Dimethyl-4-(4'-trifluormethylphenoxy)-phenyl-N'-t.-butyl-harnstoff in 25 ml Dimethylformamid werden 3,1 g Methyljodid bei einer Temperatur von 50°C langsam zugetropft, und das Reaktionsgemisch wird anschliessend während 6 Std. bei Raumtemperatur gerührt. Die erhaltene klare gelbe Reaktionslösung wird in eine Lösung von 300 ml Wasser und 50 ml 15%iger Sodalösung eingerührt und die ölige Abscheidung mit Methylenchlorid extrahiert. Die organische Phase wird mit Wasser gewaschen, über $Na_2SO_4$ getrocknet, eingedampft und der Rückstand am Hochvakuum getrocknet.

Auf diese Weise erhält man die Titelverbindung der Formel

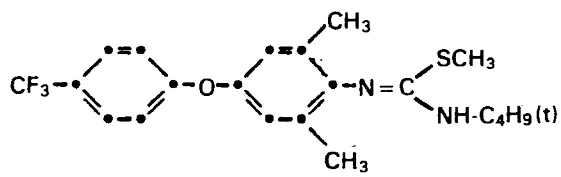

als weisse Kristalle mit einem Smp. 68-70°C.

Die folgenden Verbindungen der Formel I werden analog dem vorstehend beschriebenen Verfahren hergestellt:

| $R_1$ | $R_2$ | $R_3$ | $R_4$ | $R_5$ | $R_6$ | $R_7$ | physikalische Daten |
|---|---|---|---|---|---|---|---|
| H | H | H | $-CH_3$ | $-CH_3$ | $-CH_3$ | $-C(CH_3)_3$ | $n_D^{20}$ = 1,5832 |
| H | H | H | $-CH_3$ | $-CH_3$ | $-nC_4H_9$ | $-C(CH_3)_3$ | $n_D^{20}$ = 1,5681 |
| H | H | H | $-CH_3$ | $-CH_3$ | $-CH_3$ | $-CH(CH_3)_2$ | $n_D^{20}$ = 1,5910 |
| H | H | H | $-CH_3$ | $-CH_3$ | $-CH_3$ | $-CH_3$ | Smp. 65-67°C |
| H | H | H | $-CH_3$ | $-CH_3$ | $-CH_3$ | $-C(CH_3)_3$ | 1) Smp. (Zers.) 183°C |
| H | H | H | $-CH_3$ | $-CH_3$ | $-C_2H_5$ | $-C(CH_3)_3$ | Smp. 71-73°C |
| H | H | H | $-CH_3$ | $-CH_3$ | $-nC_3H_7$ | $-C(CH_3)_3$ | Smp. 67-69°C |
| H | H | H | $-CH_3$ | $-CH_3$ | $-CH_3$ | $-CH(CH_3)-C_2H_5$ | Smp. 58-60°C |
| 4-$CH_3$ | H | H | $-CH_3$ | $-CH_3$ | $-CH_3$ | $-C(CH_3)_3$ | Smp. 103-104°C |
| 4-$OCH_3$ | H | H | $-CH_3$ | $-CH_3$ | $-CH_3$ | $-C(CH_3)_3$ | Smp. 84-85°C |
| 4-$OCH_3$ | H | H | $-CH_3$ | $-CH_3$ | $-CH_3$ | $-CH(CH_3)_2$ | Smp. 55-57°C |
| 4-Cl | H | H | $-CH_3$ | $-CH_3$ | $-CH_3$ | $-C(CH_3)_3$ | Smp. 95-97°C |
| 4-Cl | H | H | $-CH_3$ | $-CH_3$ | $-C_2H_5$ | $-C(CH_3)_3$ | Smp. 79-80°C |
| 4-Cl | H | H | $-CH_3$ | $-CH_3$ | $-C_2H_5$ | $-C(CH_3)_3$ | 1) Smp. (Zers.) 178-179°C |
| 4-Cl | H | H | $-CH_3$ | $-CH_3$ | $-nC_3H_7$ | $-C(CH_3)_3$ | Smp. 46-48°C |
| 4-Cl | H | H | $-CH_3$ | $-CH_3$ | $-nC_4H_9$ | $-C(CH_3)_3$ | $n_D^{20}$ = 1,5725 |
| 4-Cl | H | H | $-CH_3$ | $-CH_3$ | $-nC_6H_{13}$ | $-C(CH_3)_3$ | $n_D^{20}$ = 1,5639 |
| 4-Cl | H | H | $-CH_3$ | $-CH_3$ | $-CH_3$ | $-CH(CH_3)_2$ | $n_D^{20}$ = 1,5730 |
| 4-Cl | H | H | $-CH_3$ | $-CH_3$ | $-C_2H_5$ | $-CH(CH_3)_2$ | $n_D^{20}$ = 1,5700 |
| 4-$CF_3$ | H | H | $-CH_3$ | $-CH_3$ | $-CH_3$ | $-C(CH_3)_3$ | 2) Smp. 126-127°C |
| 4-$CF_3$ | H | H | $-CH_3$ | $-CH_3$ | $-nC_3H_7$ | $-C(CH_3)_3$ | Smp. 53-55°C |

1) Salz mit 1 Mol HJ
2) Salz mit 1 Mol HOOC - COOH

| $R_1$ | $R_2$ | $R_3$ | $R_4$ | $R_5$ | $R_6$ | $R_7$ | physikalische Daten |
|---|---|---|---|---|---|---|---|
| $4-CF_3$ | H | H | $-CH_3$ | $-CH_3$ | $-C_2H_5$ | $-C(CH_3)_3$ | Smp. 71-72°C |
| $4-CF_3$ | H | H | $-CH_3$ | $-CH_3$ | $-CH_2-CH=CH_2$ | $-C(CH_3)_3$ | Smp. 78-79°C |
| $4-CF_3$ | H | H | $-CH_3$ | $-CH_3$ | $-CH_2-C\equiv CH$ | $-C(CH_3)_3$ | 1)Smp.(Zers.)170°C |
| 4-Br | H | H | $-CH_3$ | $-CH_3$ | $-CH_3$ | $-C(CH_3)_3$ | 2)Smp.(Zers.)187°C |
| $4-CF_3$ | H | H | $-CH_3$ | $-CH_3$ | $-CH_3$ | $-nC_{12}H_{25}$ | $n_D^{20}$ = 1,5288 |
| $4-CF_3$ | H | H | $-CH_3$ | $-CH_3$ | $-CH_3$ | $-C(CH_3)_3$ | 1)Smp.(Zers.)201°C |
| $4-CF_3$ | H | H | $-CH_3$ | $-CH_3$ | $-CH_3$ | $-C(CH_3)_3$ | 3)Smp.(Zers.)183°C |
| $4-CF_3$ | H | H | $-CH_3$ | $-CH_3$ | $-CH_3$ | $-CH_2-C(CH_3)_3$ | $n_D^{20}$ = 1,5439 |
| $4-CF_3$ | H | H | $-CH_3$ | $-CH_3$ | $-CH_3$ | $-CH_3$ | Smp. 36-38°C |
| $4-CF_3$ | H | H | $-CH_3$ | $-CH_3$ | $-CH_3$ | $-CH_2-CH(CH_3)_2$ | $n_D^{20}$ = 1,5465 |
| $4-CF_3$ | H | H | $-CH_3$ | $-CH_3$ | $-CH_3$ | $-nC_6H_{13}$ | $n_D^{20}$ = 1,5429 |
| $4-CF_3$ | H | H | $-CH_3$ | $-CH_3$ | $-CH_3$ | 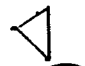 | Smp. 73-74°C |
| $4-CF_3$ | H | H | $-CH_3$ | $-CH_3$ | $-CH_3$ | 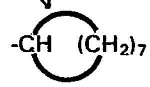 | $n_D^{20}$ = 1,5570 |
| $3-CF_3$ | H | H | $-CH_3$ | $-CH_3$ | $-CH_3$ | $-C(CH_3)_3$ | Smp. 77-78°C |
| $3-CF_3$ | H | H | $-CH_3$ | $-CH_3$ | $-CH_3$ | $-C(CH_3)_3$ | 3)Smp.(Zers.)143°C |
| 4-Cl | 3-Cl | H | $-CH_3$ | $-CH_3$ | $-CH_3$ | $-C(CH_3)_3$ | Smp. 73-75°C |
| 4-Cl | 3-Cl | H | $-CH_3$ | $-CH_3$ | $-C_2H_5$ | $-C(CH_3)_3$ | Smp. 94-96°c |
| 4-Cl | 3-Cl | H | $-CH_3$ | $-CH_3$ | $-CH_3$ | $-CH(CH_3)-C_2H_5$ | $n_D^{20}$ = 1,5954 |
| 4-Cl | H | H | $-CH_3$ | $-CH_3$ | $-CH_2-CH(CH_3)_2$ | $-C(CH_3)_3$ | Smp. 60-62°C |
| $4-CF_3$ | 2-Cl | H | $-CH_3$ | $-CH_3$ | $-CH_3$ | $-C(CH_3)_3$ | Smp. 64-66°C |
| $4-CF_3$ | 2-Cl | H | $-CH_3$ | $-CH_3$ | $-CH_3$ | $-C(CH_3)_3$ | 3)Smp.(Zers.)205°C |
| $4-CF_3$ | 2-Cl | H | $-CH_3$ | $-CH_3$ | $-CH_3$ | 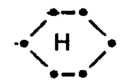 | Smp. 104-106°C |
| $4-NO_2$ | $2-CF_3$ | H | $-CH_3$ | $-CH_3$ | $-CH_3$ | $-C(CH_3)_3$ | 3)Smp.(Zers.)195°C |

1) Salz mit 1 Mol $CH_3$- 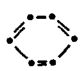 $-SO_3H$

2) Salz mit 1 Mol HJ

3) Salz mit 1 Mol 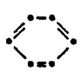 $-SO_3H$

| $R_1$ | $R_2$ | $R_3$ | $R_4$ | $R_5$ | $R_6$ | $R_7$ | physikalische Daten |
|---|---|---|---|---|---|---|---|
| 4-$CF_3$ | H | H | -$CH_3$ | H | -$CH_3$ | -$C(CH_3)_3$ | Smp. 61-63°C |
| 4-$CF_3$ | H | H | H | -$CH(CH_3)_2$ | -$CH_3$ | -$C(CH_3)_3$ | $n_D^{20}$ = 1,5459 |
| 4-$CF_3$ | H | H | H | -$CH(CH_3)_2$ | -$CH_3$ | -$C(CH_3)_3$ | 1) Smp. 94-96°C |
| 4-Cl | H | H | -$CH_3$ | -$CH(CH_3)_2$ | -$CH_3$ | -$C(CH_3)_3$ | Smp. 99-100°C |
| 4-Cl | H | H | -$CH_3$ | -$CH(CH_3)_2$ | -$C_2H_5$ | 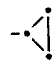 | Smp. 86-90°C |
| 4-Cl | H | H | -$CH_3$ | -$CH(CH_3)_2$ | -$CH_3$ | -$C(CH_3)_3$ | $n_D^{40}$ = 1,5708 |
| 4-Cl | H | H | -$CH_3$ | -$CH(CH_3)_2$ | -$CH_2$-C≡CH | -$C(CH_3)_3$ | Smp. 90-93°C |
| 4-Cl | H | H | -$CH_3$ | -$CH(CH_3)_2$ | -$CH_3$ | 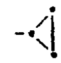 | $n_D^{40}$ = 1,5850 |
| 4-$CF_3$ | 2-Cl | H | -$CH_3$ | H | -$CH_3$ | -$C(CH_3)_3$ | |
| 4-$CF_3$ | 2-$NO_2$ | H | -$CH_3$ | -$CH_3$ | -$CH_3$ | -$C(CH_3)_3$ | |
| 4-$CF_3$ | H | H | -$CH_3$ | -$CH_3$ | -$CH_2O$-$CH_3$ | -$C(CH_3)_3$ | |
| 4-$CF_3$ | H | H | -$CH_3$ | -$CH_3$ | -$CH_2$-S-$CH_3$ | -$C(CH_3)_3$ | |
| 4-$CF_3$ | H | H | -$CH_3$ | -$CH_3$ | -$CH_3$ | -$C(CH_3)_2$-$C_2H_5$ | |
| 4-$CF_3$ | H | H | -$CH_3$ | -$CH_3$ | -$CH_3$ | -$CH(C_2H_5)_2$ | |
| 4-$CF_3$ | H | H | -$CH_3$ | -$CH_3$ | -$CH_3$ | -$CH(CH_3)$-$C_3H_7$ | |
| 4-$CF_3$ | H | H | -$CH_3$ | -$CH_3$ | -$CH_3$ | -$nC_8H_{17}$ | |
| 4-$CF_3$ | H | H | -$CH_3$ | -$CH_3$ | -$CH_3$ | -$CH_2$-CH=$CH_2$ | |
| 4-$CF_3$ | H | H | -$CH_3$ | -$CH_3$ | -$CH_3$ | 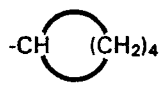 | |
| 4-$CF_3$ | H | H | -$CH_3$ | -$CH_3$ | 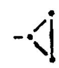 | -$C(CH_3)_3$ | |
| 4-$CF_3$ | H | H | -$CH_3$ | -$CH_3$ | 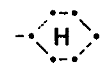 | -$C(CH_3)_3$ | |

1) Salz mit 1 Mol HJ

*Beispiel 2*

*Herstellung von N-2,6-Dimethyl-4-(4'-trifluormethylphenoxy)-phenyl-N'-t.-butyl-thioharnstoff (Ausgangsstoff für Beispiel 1):*

16,1 g 2,6-Dimethyl-4-(4'-trifluormethylphenoxy)-phenylisothiocyanat und 20 g t.-Butylamin werden 2 Stunden bei 40°C und anschliessend während 5 Stunden bei Raumtemperatur verrührt.

Nach dem Eindampfen der erhaltenen Reaktionslösung wird der Rückstand in Methylenchlorid aufgenommen, mit ~5%iger Salzsäure neutralisiert und mit wenig Wasser gewaschen. Nach dem Trocknen über Natriumsulfat wird die Methylenchloridlösung stark eingeengt und durch Zugabe von Hexan zum Kristallisieren gebracht. Die Kristalle werden abgenutscht, abgepresst und getrocknet. Auf diese Weise erhält man die Titelverbindung der Formel

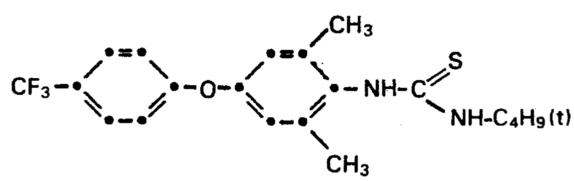

als ein hellbeiges Pulver mit einem Smp. von 118 bis 120°C.

Die folgenden Verbindungen der Formel II werden in analoger Weise erhalten:

| $R_1$ | $R_2$ | $R_3$ | $R_4$ | $R_5$ | $R_7$ | physikalische Daten |
|---|---|---|---|---|---|---|
| H | H | H | $-CH_3$ | $-CH_3$ | $-C(CH_3)_3$ | Smp. 161-163°C |
| H | H | H | $-CH_3$ | $-CH_3$ | $-CH(CH_3)_2$ | Smp. 129-131°C |
| H | H | H | $-CH_3$ | $-CH_3$ | $-CH_3$ | Smp. 156-158°C |
| H | H | H | $-CH_3$ | $-CH_3$ | $-CH-C_2H_5$<br>$CH_3$ | Smp. 113-114°C |
| 4-$CH_3$ | H | H | $-CH_3$ | $-CH_3$ | $-C(CH_3)_3$ | Smp. 148-150°C |
| 4-$OCH_3$ | H | H | $-CH_3$ | $-CH_3$ | $-C(CH_3)_3$ | Smp. 130-132°C |
| 4-$OCH_3$ | H | H | $-CH_3$ | $-CH_3$ | $-CH(CH_3)_2$ | Smp. 154-156°C |
| 4-Cl | H | H | $-CH_3$ | $-CH_3$ | $-C(CH_3)_3$ | Smp. 128-129°C |
| 4-Cl | H | H | $-CH_3$ | $-CH_3$ | $-CH(CH_3)_2$ | Smp. 148-151°C |
| 4-$CF_3$ | H | H | $-CH_3$ | $-CH_3$ | $-CH_3$ | Smp. 160-162°C |
| 4-$CF_3$ | H | H | $-CH_3$ | $-CH_3$ | $-C(CH_3)_3$ | Smp. 118-119°C |
| 4-Br | H | H | $-CH_3$ | $-CH_3$ | $-C(CH_3)_3$ | Smp. 137-139°C |
| 4-$CF_3$ | H | H | $-CH_3$ | $-CH_3$ | $-nC_{12}H_{25}$ | $n_D^{20} = 1,5402$ |
| 4-$CF_3$ | H | H | $-CH_3$ | $-CH_3$ | $-CH_2-C(CH_3)_3$ | Smp. 105-107°C |
| 4-$CF_3$ | H | H | $-CH_3$ | $-CH_3$ | $-CH_2-CH(CH_3)_2$ | Smp. 115-116°C |
| 4-$CF_3$ | H | H | $-CH_3$ | $-CH_3$ | $-nC_6H_{13}$ | Smp. 111-113°C |
| 4-$CF_3$ | H | H | $-CH_3$ | $-CH_3$ | 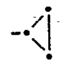 | Smp. 130-132°C |
| 4-$CF_3$ | H | H | $-CH_3$ | $-CH_3$ | 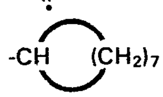 | Smp. 122-124°C |
| 3-$CF_3$ | H | H | $-CH_3$ | $-CH_3$ | $-C(CH_3)_3$ | Smp. 114-116°C |
| 3-$CF_3$ | H | H | $-CH_3$ | $-CH_3$ | $-CH_2-C(CH_3)_3$ | hochviskose Masse |
| 4-Cl | 3-Cl | H | $-CH_3$ | $-CH_3$ | $-C(CH_3)_3$ | Smp. 124-125°C |
| 4-Cl | 3-Cl | H | $-CH_3$ | $-CH_3$ | $-CH-C_2H_5$<br>$CH_3$ | Smp. 97-99°C |
| 4-$CF_3$ | 2-Cl | H | $-CH_3$ | $-CH_3$ | $-C(CH_3)_3$ | Smp. 136-137°C |
| 4-$CF_3$ | 2-Cl | H | $-CH_3$ | $-CH_3$ | 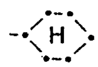 | Smp. 129-130°C |
| 4-$NO_2$ | 2-$CF_3$ | H | $-CH_3$ | $-CH_3$ | $-C(CH_3)_3$ | Smp. 135-136°C |
| 4-$CF_3$ | H | H | $-CH_3$ | H | $-C(CH_3)_3$ | Smp. 122-124°C |
| 4-$CF_3$ | H | H | $-CH_3$ | H | $-CH_3$ | Smp. 146-148°C |
| 4-$CF_3$ | H | H | H | $-CH(CH_3)_2$ | $-C(CH_3)_3$ | Smp. 125-127°C |
| 4-$CF_3$ | H | H | H | $-CH(CH_3)_2$ | $-CH_3$ | Smp. 180-182°C |
| 4-$CF_3$ | H | H | H | $-CH(CH_3)_2$ | 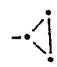 | Smp. 116-118°C |
| 4-Cl | H | H | $-CH_3$ | $-CH(CH_3)_2$ | 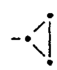 | Smp. 143-145°C |
| 4-Cl | H | H | $-CH_3$ | $-CH(CH_3)_2$ | $-C(CH_3)_3$ | Smp. 65°C |
| 4-$CF_3$ | 2-Cl | H | $-CH_3$ | H | $-C(CH_3)_3$ | |
| 4-$CF_3$ | 2-$NO_2$ | H | $-CH_3$ | $-CH_3$ | $-C(CH_3)_3$ | |
| 4-$CF_3$ | H | H | $-CH_3$ | $-CH_3$ | $-C(CH_3)_3$ | |
| 4-$CF_3$ | H | H | $-CH_3$ | $-CH_3$ | $-C(CH_3)_2-C_2H_5$ | |
| 4-$CF_3$ | H | H | $-CH_3$ | $-CH_3$ | $-CH(CH_2H_5)_2$ | |
| 4-$CF_3$ | H | H | $-CH_3$ | $-CH_3$ | $-CH(CH_3)-C_3H_7$ | |
| 4-$CF_3$ | H | H | $-CH_3$ | $-CH_3$ | $-nC_8H_{17}$ | |
| 4-$CF_3$ | H | H | $-CH_3$ | $-CH_3$ | $-CH_2-CH=CH_2$ | |
| 4-$CF_3$ | H | H | $-CH_3$ | $-CH_3$ | 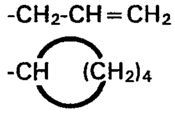 | |

*Beispiel 3*

*Insektizide Frassgift-Wirkung: Spodoptera littoralis, Dysdercus fasciatus und Heliothis virescens*

Baumwollpflanzen wurden mit einer wässrigen Emulsion enthaltend 0,05% der zu prüfenden Verbindung (erhalten aus einem 10%igen emulgierbaren Konzentrat) besprüht.

Nach dem Antrocknen des Belages wurden die Pflanzen mit Larven der Spezies Spodoptera littoralis (L3-Stadium) Dysdercus fasciatus (L4) oder Heliothis virescens (L3) besetzt. man verwendete pro Versuchsverbindung und pro Test-Spezies zwei Pflanzen, und die Auswertung der erzielten Abtötungsraten erfolgte nach 2, 4, 24 und 48 Stunden. Der Versuch wurde bei 24°C und 60% relativer Luftfeuchtigkeit durchgeführt.

Verbindung der Formeln I und II gemäss Beispiel 1 und 2 zeigen im obigen Versuch gute Wirkung gegen Larven der Spezies Spodoptera littoralis, Dysdercus fasciatus und Heliothis virescens.

*Beispiel 4*

*Insektizide Frassgift-Wirkung: Leptinotarsa decemlineata*

Bei gleicher Arbeitsweise unter Verwendung von Larven der Spezies Leptinotarsa decemlineata (L3) und Kartoffelpflanzen anstelle von Baumwollpflanzen wurde die im Beispiel 3 beschriebene Versuchsmethode wiederholt.

In diesem Versuch ergaben Verbindungen der Formel I und II gemäss Beispiel 1 und 2 gute Wirkung gegen Larven der Spezies Leptinotarsa decemlineata.

*Beispiel 5*

*Wirkung gegen pflanzenschädigende Akariden: Tetranychus urticae (OP-sensible) und Tetranychus cinnabarinus (OP-tolerant)*

Die Primärblätter von Phaseolus Vulgaris-Pflanzen wurden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt. (Die Toleranz bezieht sich auf die Verträglichkeit gegen Diazinon).

Die so behandelten infestierten Pflanzen wurden mit einer Versuchslösung enthaltend 400 oder 200 ppm der zu prüfenden Verbindung bis zur Tropfnässe besprüht.

Nach 24 Stunden und wiederum nach 7 Tagen wurden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25°C.

Verbindungen der Formeln I und II gemäss Beispiel 1 und 2 zeigten in diesem Versuch gute Wirkung gegen Tetranychus urticae und Tetranychus cinnabarinus.

*Beispiel 6*

*Wirkung gegen ektoparasitäre Akariden (Zecken) Rhipicephalus bursa (Imagines und Larven), Amblyomma hebraeum (♀ Imagines, Nymphen und Larven)* und Boophilus microplus (Larven - OP-sensible und OP-tolerant)

Als Testtiere wurden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die Testtiere wurden für kurze Zeit in eine wässrige Emulsion bzw. Lösung enthaltend 0,1; 1,0; 10; 50 oder 100 ppm der zu prüfenden Verbindung getaucht.

Die in Teströhrchen befindlichen Emulsionen bzw. Lösungen wurden dann mit Watte aufgenommen und die benetzten Testtiere in den so kontaminierten Röhrchen belassen.

Eine Auswertung der erzielten Abtötungsrate bei jeder Konzentration erfolgte für Larven nach 3 Tagen und für Nymphen und Imagines nach 14 Tagen.

Verbindungen der Formeln I und II gemäss Beispiel 1 und 2 zeigten in diesem Versuch gute Wirkung gegen Larven, Nymphen und Imagines der Spezies Rhipicephalus bursa und Amblyomma hebraeum sowie gegen Larven (OP-res. und OP-sens.) der Spezies Boophilus microplus.

*Beispiel 7*

*Pflanzenmitizide Blattpenetrations-Wirkung auf Tetranychus cinnabarinus und Panonychus citri*

Es wurden für den Versuch eingetopfte Buschbohnenpflanzen, die von Tetranychus cinnabarinus befallen waren, und eingetropfte Citruspflanzen, die von Panonychus citri befallen waren, verwendet.

Die Blattoberseiten der mit diesen Milben infestierten Versuchspflanzen wurden mit einer Emulsionszubereitung enthaltend 400 ppm des zu prüfenden Wirkstoffes besprüht. Nach dem Antrocknen des Sprühbelages wurde jeweils der Rand der Blattoberseite einer Anzahl befallener Blätter mit einem Wulst aus zähflüssigem Leim (Pomona-Raupenleim) abgegrenzt, um ein Überlaufen der Milben von der Blattunterseite auf die Blattoberseite zu verhindern.

Die behandelten Pflanzen wurden dann im Gewächshaus bei einer Temperatur von 25° bis 29°C gehalten. Fünf Tage nach der Wirkstoffapplikation wurde festgestellt, ob eine translaminare Wirkung, d.h. Blattpenetration des Wirkstoffs, eingetreten war, und zwar durch Bestimmung der Mortalität der postembryonalen und adulten Stadien.

Verbindungen der Formeln I und II gemäss den vorstehenden Beispielen 1 und 2 zeigten gute Wirkung in obigem Test.

**Patentansprüche**

1. Verbindung der Formel I

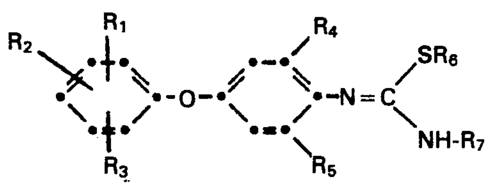

(I)

worin $R_1$, $R_2$ und $R_3$ unabhängig voneinander Wasserstoff, $C_1$-$C_4$-Alkyl, Methoxy, Halogen. Trifluormethyl oder Nitro, $R_4$ Wasserstoff oder Methyl, $R_5$ $C_1$-$C_3$-Alkyl, $R_6$ $C_1$-$C_6$-Alkyl, $C_2$-$C_6$-Alkenyl, $C_3$-$C_6$-Alkinyl, $C_3$-$C_6$-Cycloalkyl, $C_2$-$C_4$-(Alkoxyalkyl) oder $C_2$-$C_4$-(Alkylthioalkyl) und $R_7$ $C_1$-$C_{12}$-Alkyl, $C_3$-$C_6$-Alkenyl oder $C_3$-$C_6$-Cycloalkyl bedeuten, und deren Säureadditionssalze.

2. Verbindung nach Anspruch 1 der Formel I, worin $R_1$ und $R_2$ unabhängig voneinander Wassertoff, Methyl, Chlor, Trifluormethyl, Methoxy oder Nitro, $R_3$ Wasserstoff, $R_4$ Wasserstoff oder Methyl und $R_5$ Methyl bedeuten.

3. Verbindung nach Anspruch 1 oder 2 der Formel I, worin $R_2$ Wasserstoff oder Chlor bedeutet.

4. Verbindung nach Anspruch 1 bis 3 der Formel I, worin sich der Rest $R_1$ in 4-Stellung befindet.

5. Verbindung nach Anspruch 4 der Formel I, worin $R_1$ Trifluormethyl oder Chlor bedeutet.

6. Verbindung nach Anspruch 1 bis 5 der Formel I, worin $R_6$ und $R_7$ $C_1$-$C_4$-Alkyl bedeuten.

7. Verbindung nach Anspruch 6 der Formel I, worin $R_6$ Methyl oder Äthyl und $R_7$ tert.-Butyl bedeuten.

8. Verbindung nach Anspruch 7 der Formel

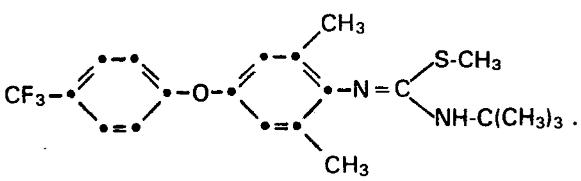

9. Verbindung nach Anspruch 7 der Formel

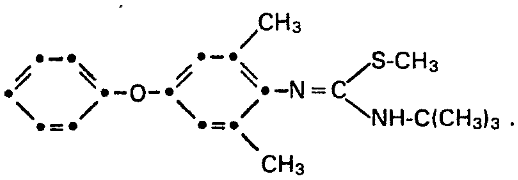

10. Verbindung nach Anspruch 7 der Formel

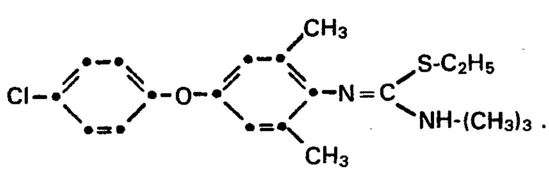

11. Verbindung nach Anspruch 7 der Formel

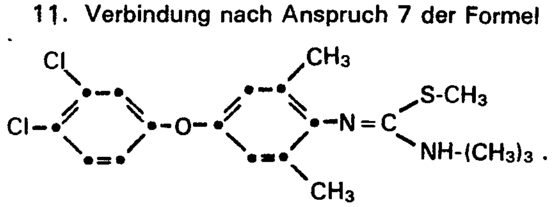

12. Verbindung nach Anspruch 7 der Formel

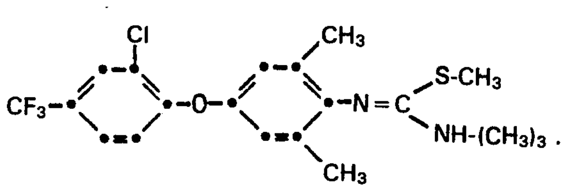

13. Verfahren zur Herstellung einer Verbindung gemäss einem der Ansprüche 1 bis 12, dadurch gekennzeichnet, dass man eine Verbindung der Formel II

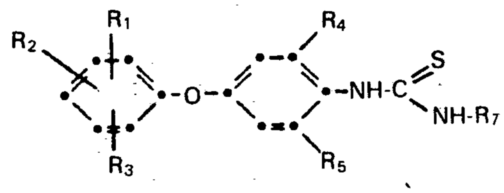

(II)

mit einer Verbindung der Formel III

$R_6$ - Hal (III)

umsetzt, worin $R_1$ bis $R_7$ die in den Ansprüchen 1 bis 7 angegebenen Bedeutungen haben und «Hal» für ein Halogenatom steht.

14. Verbindung der Formel II

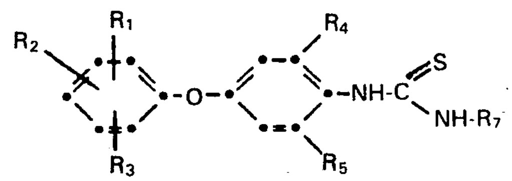

(II)

worin $R_1$, $R_2$, $R_3$, $R_4$, $R_5$ und $R_7$ die entsprechenden, jeweils in einem der Ansprüche 1 bis 7 angegebenen Bedeutungen haben.

15 Verbindung nach Anspruch 14 der Formel

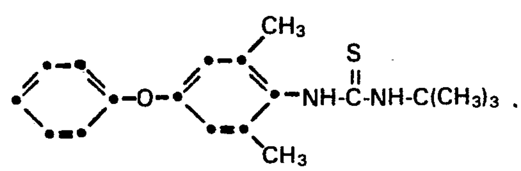

16 Verbindung nach Anspruch 14 der Formel

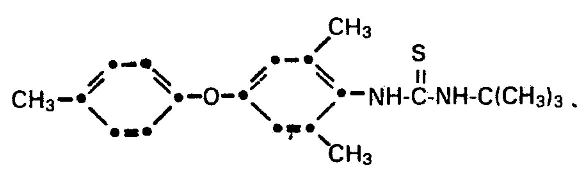

17. Verbindung nach Anspruch 14 der Formel

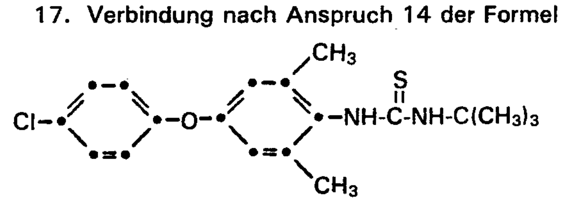

18. Verbindung nach Anspruch 14 der Formel

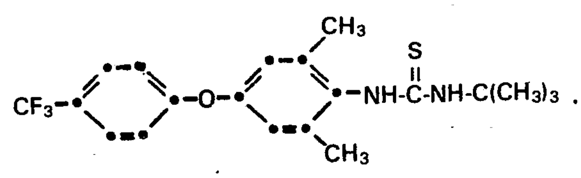

19. Verbindung nach Anspruch 14 der Formel

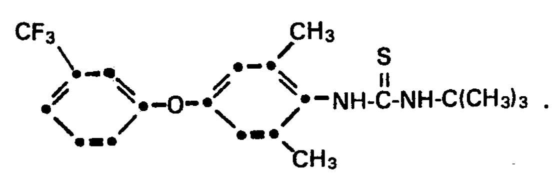

20. Verbindung nach Anspruch 14 der Formel

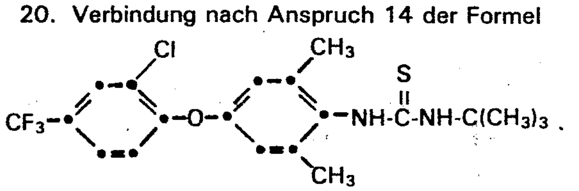

21. Verfahren zur Herstellung einer Verbindung nach einem der Ansprüche 14 bis 20, dadurch gekennzeichnet, dass man eine Verbindung der Formel IV

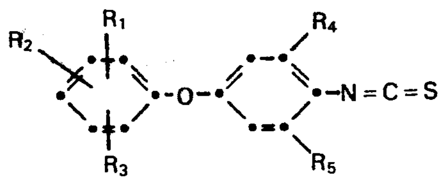

(IV)

mit einer Verbindung der Formel V

$R_7$ - $NH_2$ (V)

umsetzt, worin $R_1$ bis $R_5$ und $R_7$ die entsprechenden jeweils in einem der Ansprüche 1 bis 7 angegebenen Bedeutungen haben.

22. Schädlingsbekämpfungsmittel enthaltend als aktive Komponente eine der in einem der Ansprüche 1 bis 12 bzw. 14 bis 20 definierten Verbindungen.

23. Verwendung einer der in einem der Ansprüche 1 bis 12 bzw. 14 bis 20 definierten Verbindungen zur Bekämpfung von Vertretern der Ordnung Akarina und von Insekten.

24. Verwendung nach Anspruch 23 zur Bekämpfung von pflanzenschädigenden Spinnmilben und Insekten.

## Claims

1. A compound of the formula I

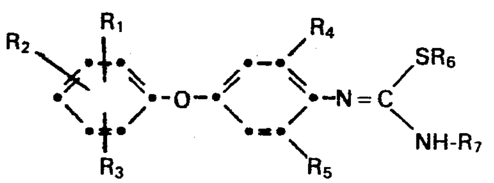

(I)

wherein $R_1$, $R_2$ and $R_3$ independently of one another are each hydrogen, $C_1$-$C_4$-alkyl, methoxy, halogen, trifluoromethyl or nitro, $R_4$ is hydrogen or methyl, $R_5$ is $C_1$-$C_3$-alkyl, $R_6$ is $C_1$-$C_6$-alkyl, $C_2$-$C_6$-alkenyl, $C_3$-$C_5$-alkynyl, $C_3$-$C_6$-cycloalkyl, $C_2$-$C_4$-(alkoxyalkyl) or $C_2$-$C_4$-(alkylthioalkyl), and $R_7$ is $C_1$-$C_{12}$-alkyl, $C_3$-$C_5$-alkenyl or $C_3$-$C_8$-cycloalkyl, and acid addition salts thereof.

2. A compound of the formula I according to claim 1, wherein $R_1$ and $R_2$ independently of one another are each hydrogen, methyl, chlorine, trifluoromethyl, methoxy or nitro, $R_3$ is hydrogen, $R_4$ is hydrogen or methyl, and $R_5$ is methyl.

3. A compound of the formula I according to claim 1 or 2, wherein $R_2$ is hydrogen or chlorine.

4. A compound of the formula I according to any one of claims 1 to 3, wherein the radical $R_1$ is in the 4-position.

5. A compound of the formula I according to claim 4, wherein $R_1$ is trifluormethyl or chlorine.

6. A compound of the formula I according to any one of claims 1 to 5, wherein $R_6$ and $R_7$ are each $C_1$-$C_4$-alkyl.

7. A compound of the formula I according to claim 6, wherein $R_6$ is methyl or ethyl, and $R_7$ is tert-butyl.

8. A compound according to claim 7 of the formula

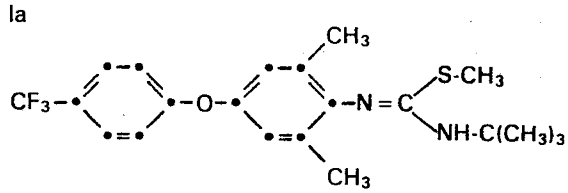

9. A compound according to claim 7 of the formula

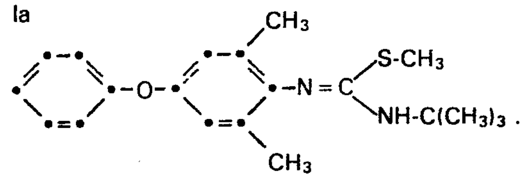

10. A compound according to claim 7 of the formula

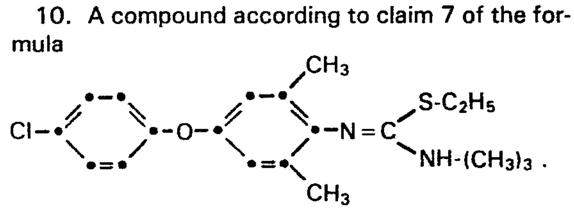

11. A compound according to claim 7 of the formula

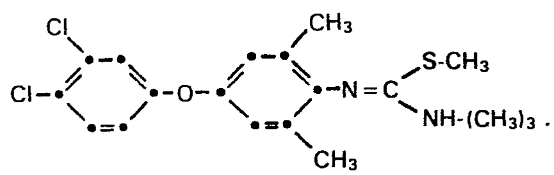

12. A compound according to claim 7 of the formula

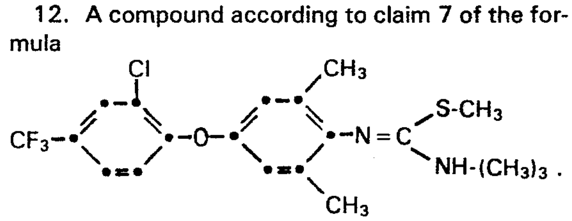

13. A process for producing a compound according to any one of claims 1 to 12 inclusive. which process comprises reacting a compound of the formula II

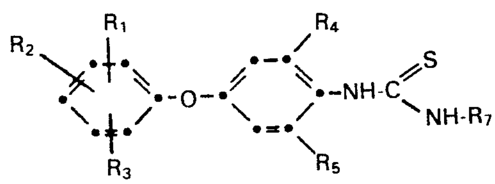

(II)

with a compound of the formula III

$R_6$ - Hal (III)

wherein $R_1$ to $R_7$ have the meanings given in claims 1 to 7, and «Hal» is a halogen atom.

14. A compound of the formula II

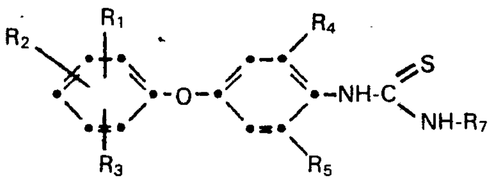

(II)

wherein $R_1$, $R_2$, $R_3$, $R_4$, $R_5$ and $R_7$ each have the corresponding meanings given in any one of claims 1 to 7 inclusive.

15. A compound according to claim 14 of the formula

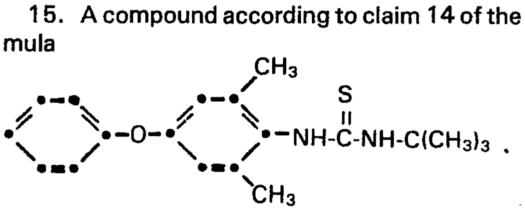

16. A compound according to claim 14 of the formula

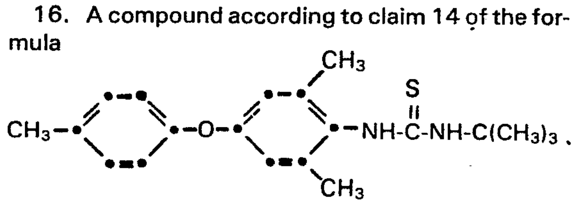

17. A compound according to claim 14 of the formula

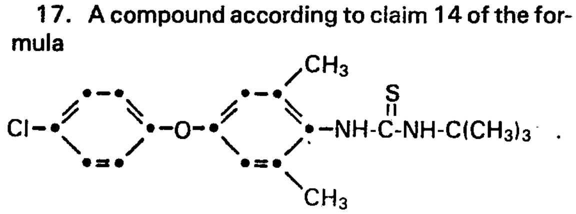

18. A compound according to claim 14 of the formula

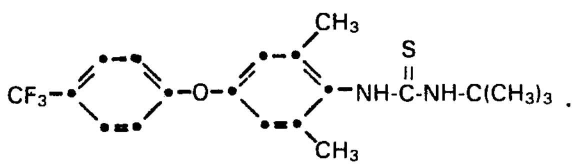

19. A compound according to claim 14 of the formula

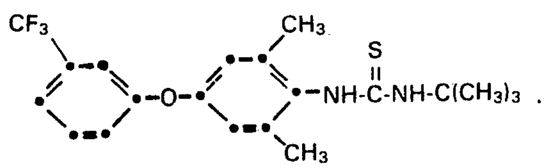

20. A compound according to claim 14 of the formula

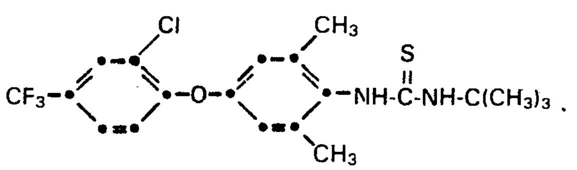

21. A process for producing a compound according to any one of claims 14 to 20, which process comprises reacting a compound of the formula IV

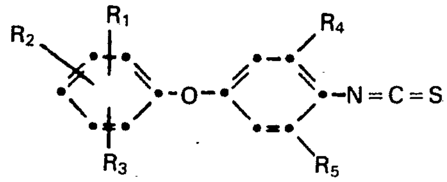

(IV)

with a compound of the formula V

$R_7 - NH_2$ (V)

wherein $R_1$ to $R_5$ and $R_7$ each have the corresponding meanings given in any one of claims 1 to 7 inclusive.

22. A pesticidal composition containing as active ingredients one of the compounds defined in claims 1 to 12 and 14 to 20 inclusive.

23. Use of any one of the compounds defined in any one of claims 1 to 12 and 14 to 20 inclusive for combating representatives of the order Acarina, and insects.

24. Use according to claim 23 for combating plant-damaging spider mites and insects.

**Revendications**

1 Composé de formule I

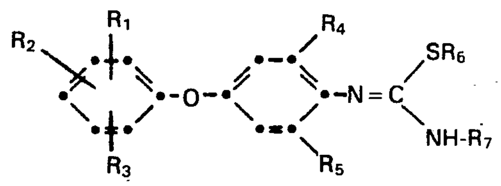

(I)

dans laquelle $R_1$, $R_2$ et $R_3$ représentent indépendamment, l'un de l'autre l'hydrogène, un groupe alkyle en C1-C4, méthoxy, un halogène, un groupe trifluorméthyle ou nitro, $R_4$ représente l'hydrogène ou un groupe méthyle, $R_5$ représente un groupe alkyle en C1-C3, $R_6$ représente un groupe alkyle en C1-C6, alcényle en C2-C6, alcynyle en C3-C5, cycloalkyle en C3-C6, alcoxyalkyle en C2-C4 ou alkylthioalkyle en C2-C4 et $R_7$ représente un groupe alkyle en C1-C12, alcényle en C3-C5 ou cycloalkyle en C3-C8, et ses sels formées par addition avec des acides.

2. Composé selon la revendication 1 de formule I dans laquelle $R_1$ et $R_2$ représentent, indépendamment l'un de l'autre, l'hydrogène, un groupe méthyle, le chlore, un groupe trifluorométhyle, méthoxy ou nitro, $R_3$ représente l'hydrogène, $R_4$ représente l'hydrogène ou un groupe méthyle et $R_5$ représente un groupe méthyle.

3. Composé selon la revendication 1 ou 2 de formule I dans laquelle $R_2$ représente l'hydrogène ou le chlore.

4. Composé selon les revendications 1 à 3 de formule I dans laquelle le reste $R_1$ est en position 4.

5. Composé selon la revendication 4 de formule I dans laquelle $R_1$ représente un groupe trifluorométhyle ou le chlore.

6. Composé selon les revendications 1 à 5 de formule I dans laquelle $R_6$ et $R_7$ représentent des groupes alkyles en C1-C4.

7. Composé selon la revendication 6 de formule I dans laquelle $R_6$ représente un groupe méthyle ou éthyle et $R_7$ un groupe tert.-butyle.

8. Composé selon la revendication 7 de formule

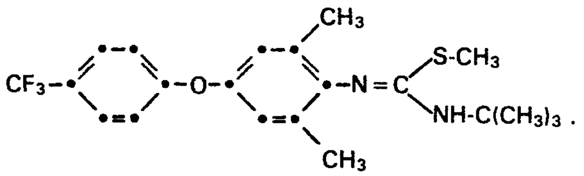

9. Composé selon la revendication 7 de formule

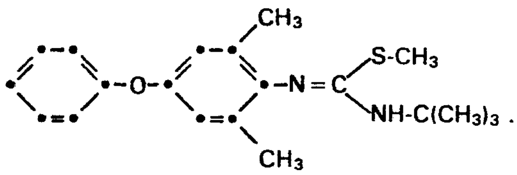

10. Composé selon la revendication 7 de formule

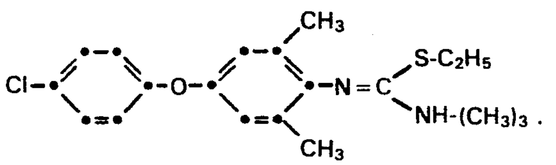

11. Composé selon la revendication 7 de formule

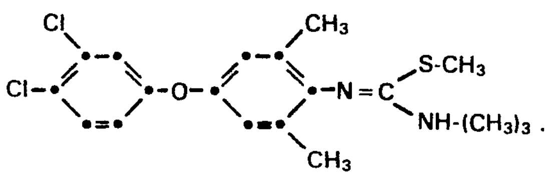

12. Composé selon la revendication 7 de formule

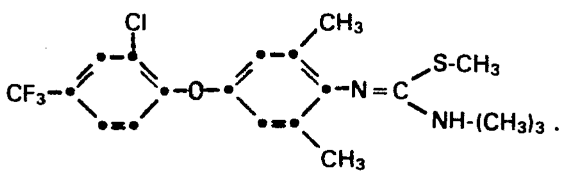

13. Procédé de préparation d'un composé selon l'une des revendications 1 à 12, caractérisé en ce que l'on fait réagir un composé de formule II

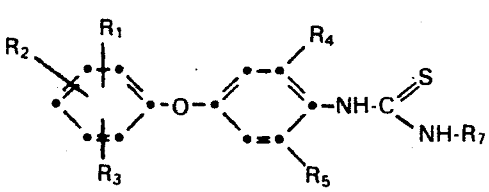 (II)

avec un composé de formule III

$R_6$ - Hal (III)

$R_1$ à $R_7$ ayant les significations indiquées dans les revendications 1 à 7 et «Hal» représentant un atome d'halogène.

14. Composé de formule II

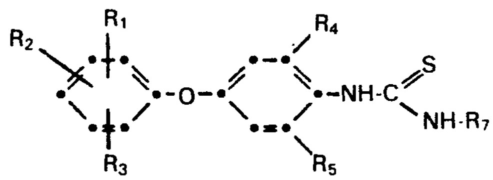 (II)

dans laquelle $R_1$, $R_2$, $R_3$, $R_4$, $R_5$ et $R_7$ ont les significations déjà indiquées dans l'une des revendications 1 à 7.

15. Composé selon la revendication 14 de formule

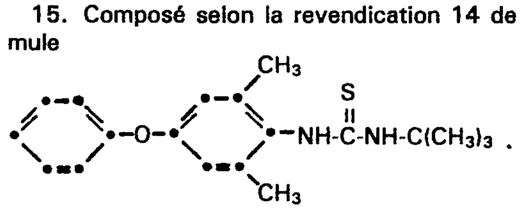

16. Composé selon la revendication 14 de formule

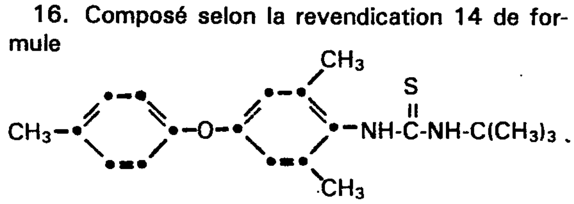

17. Composé selon la revendication 14 de formule

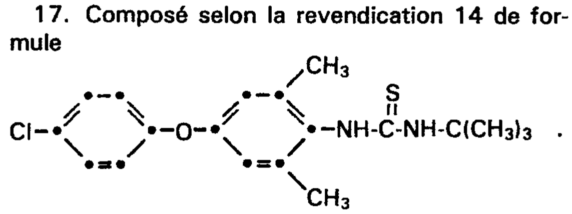

18. Composé selon la revendication 14 de formule

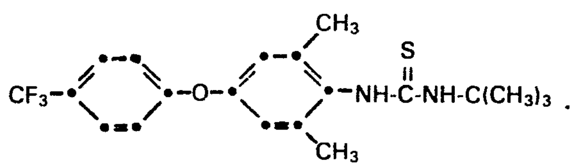

19. Composé selon la revendication 14 de formule

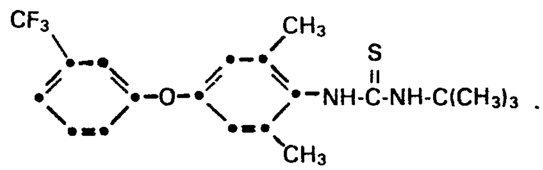

20. Composé selon la revendication 14 de formule

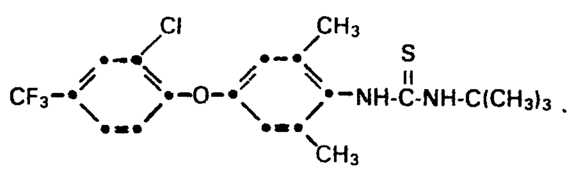

21. Procédé de préparation d'un composé selon l'une des revendications 14 à 20, caractérisé en ce que l'on fait réagir un composé de formule IV

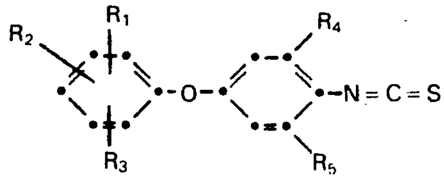

(IV)

avec un composé de formule V

$R_7 - NH_2$ (V)

$R_1$ à $R_5$ et $R_7$ ayant les significations déjà indiquées dans l'une des revendications 1 à 7.

22. Produit pesticide contenant en tant que composant actif l'un des composés définis dans l'une des revendications 1 à 12 ou 14 à 20.

23. Utilisation de l'un des composés définis dans l'une des revendications 1 à 12 ou 14 à 20 dans la lutte contre les représentants de l'ordre des acariens et les insectes.

24. Utilisation selon la revendication 23, dans la lutte contre les mites et les insectes nuisibles pour les végétaux.